# EUROPEAN PATENT SPECIFICATION

(11) **EP 1 338 628 B1**
(45) Date of publication and mention of the grant of the patent: **16.12.2009**
(21) Application number: 01978955.1
(22) Date of filing: 30.10.2001
(51) Int. Cl.: C09C 1/64, C09D 11/00, C08K 9/04

(54) **ALUMINUM PIGMENT COMPOSITION**
ALUMINIUMPIGMENTZUSAMMENSETZUNG
COMPOSITION A BASE DE PIGMENT D'ALUMINIUM

(30) Priority: 02.11.2000 JP 2000335814
(43) Date of publication of application: 27.08.2003
(73) Proprietor: Asahi Kasei Kabushiki Kaisha, Osaka-shi, Osaka 530-8205 (JP)
(72) Inventor: KATSUTA, Shigeki, Yokohama-shi, Kanagawa 234-0052 (JP)
(74) Representative: von Kreisler, Alek
(86) International application number: PCT/JP2001/009520
(87) International publication number: WO 2002/036689

(56) References cited:
- EP-A2- 0 328 906
- JP-A- 1 110 568
- JP-A- 4 045 835
- JP-A- 10 292 136
- JP-A- 11 080 587
- JP-A- 59 170 152
- US-A- 5 931 996

## Description

### TECHNICAL FIELD

The present invention relates to an aluminum pigment composition which is a pigment used mainly in coating materials for automobiles, bicycles, toys, household electric appliances, buildings, vehicles, industrial machines and their parts, etc., various inks (e.g. printing ink, writing ink, duplicating ink and specialty ink), resin compositions, ceramic products, cosmetics and the like; a process for producing said pigment composition; a coating composition, an ink composition, a resin compound and a cosmetic which are obtained by using said pigment composition; a coated product obtained by coating with the coating composition containing said pigment composition; and a print obtained by printing with the ink composition containing said pigment composition.

### BACKGROUND ART

As a method for obtaining a colored metallic coating film, methods have been known which use flaky aluminum and an organic or inorganic color pigment in combination as pigment components. These methods, however, are disadvantageous, for example, in that they require a troublesome procedure wherein two or more pigments are added, and that a color tone change is caused by a slight change in the proportions of the pigments.

The above problems have been solved by a method which incorporates pigments having both a metallic luster and a tinting strength. This method uses a metallic pigment. For example, a method has been devised which uses a colored metallic pigment or in which a desirable color is given to the metallic pigment itself. There is another method that uses a pigment having a pearly luster, which differs from a metallic luster.

For example, in a method for obtaining a golden colored metallic coating film, brass pieces can be used as a pigment. The starting brass, however, can be used in only a few cases because it is expensive and harmful to human beings. In addition, it is disadvantageous in that its color change and luster deterioration are high depending on the conditions it is exposed to. On the other hand, there is a method for giving a desirable color to the surface of an inexpensive and very safe metal in which the surface of flaky aluminum is treated with a substance capable of being colored (JP-B-53-4004, JP-A-60-50176, JP-A-60-72969, JP-B-6-92546, JP-A-7-228797, JP-A-8-85765, JP-A-1-311176, JP-A-8-209024 and JP-A-8-333602).

In detail, JP-B-53-4004 discloses a method in which colored flaky aluminum is obtained by treating flaky aluminum with boehmite, if necessary, and immersing the treated aluminum in a weakly alkaline solution containing a metal salt and an organic compound having chelating capability, to form a chelate on the surface of the flaky aluminum.

This method, however, is disadvantageous in that the flaky aluminum and the alkali react with each other to gelatinize, resulting in the production of hydrogen gas. Therefore, it is difficult to obtain a coating film with a metallic luster by said method.

JP-A-60-50176 and JP-A-60-72969 disclose methods in which golden and flaky aluminum is obtained by treating flaky aluminum with chromic acid, bichromic acid and a fluoride to adsorb hexavalent or trivalent chromium on the surface of this flaky aluminum. These methods, however, have environmental, economical and safety problems. Such as the need for antipollution measures to handle the chromium waste.

JP-B-6-92546 discloses a method in which a coloring pigment, which is easily desorbed, is protected by chemical adsorption of the coloring pigment on the surface of a metallic pigment and coating of the thus treated surface with a resin. This method is disadvantageous in that it requires a troublesome procedure and that the color of the metallic coating film obtained is not very different from that of a metallic coating film obtained by a conventional method.

JP-A-7-228797 and JP-A-8-85765 disclose methods in which aluminum powder is treated with an organotitanium compound and then a developer. These methods are intended to chemically stabilize the aluminum surface and not cause the formation of interference colors.

JP-A-1-311176 discloses a method in which the surface of an aluminum pigment is coated with titanium oxide by using titanium tetrachloride vapor and water vapor. JP-A-8-209024 discloses a method in which a pigment having interference colors is obtained by coating a flaky metal base material with multiple coating layers with different refractive indexes. In particular, JP-A-8-209024 discloses a method in which, as in the present invention, a glitter is obtained that can vary in color tone when light is cast on a coating film containing the glitter depending on the angle between the incident light and the reflected light (this property is hereinafter referred to as the color flop property). These methods, however, have the disadvantage in that they require a troublesome procedure and that the pigment tends to aggregate and results in an awkward coating material.

JP-A-8-333602 discloses a method in which a coloring pigment having interference colors is obtained by first producing flaky titanium powder and oxidizing the surface of the powder. This method has the disadvantage in that it is difficult to produce titanium flakes easily at low cost.

In addition, pigments having a pearly luster different from a metallic luster have been invented. These are thin leaves of a material having a high refractive index, or pigments obtained by forming a thin film of a material having a high refractive index on a colorless and transparent inorganic base material. The pigments of both kinds are color pigments utilizing the interference colors of the thin leaves or the thin film.

As the pigments having a pearly luster, for example, guanine crystals extracted from the scales of herrings are famous. The crystals, however, have the disadvantage in that they can be collected in only a very small amount as a natural material and hence are expensive. Crystalline pieces of basic carbonates and bismuth oxychloride have been developed but are not often used at present because they are toxic.

Accordingly, for example, JP-B-35-5367 discloses a method in which a pearly luster is given to a transparent and flaky material by coating it with titanium dioxide produced from a titanium alcoholate. JP-B-39-28885 discloses a technique for obtaining a pigment capable of giving interference colors, in which a transparent micaceous material is coated with titanium dioxide produced from titanium tetrachloride. JP-B-43-25644 discloses a technique for obtaining a pigment capable of giving interference colors, in which a metal oxide such as titanium dioxide, zirconium dioxide, iron oxide or chromium oxide is formed on transparent mica flakes.

All of such pigments having a pearly luster, however, develop interference colors owing to the formation of a layer with a high refractive index on a colorless and transparent base material as described above. They have a low hiding power as a matter of course and they are strongly affected by the substrate when formed into a coating film on the substrate. Therefore, they have the disadvantage in that a troublesome procedure should be employed. For example, the number of prime-coating operations is increased (JP-A-59-160571 and JP-A-59-215857), or the pigment having a pearly luster is used in admixture with a pigment having a high hiding power, such as flaky aluminum. Moreover, since a natural material such as mica is used as the lamellar base material, it is difficult to make the particle size distribution and shape of the lamellar base material satisfy desirable conditions, so that proper control of various design properties due to the particle size distribution and shape of the lamellar base material is difficult.

On the other hand, JP-A-1-110568 and JP-A-10-292136 disclose processes in which a coloring pigment capable of developing interference colors is produced by providing hydrolyzates of a titanium alcoholate on the surface of flaky aluminum. These processes do not give a coloring pigment having the color flop property.

JP-A-2-669 discloses a process in which a coloring pigment capable of developing interference colors is produced by hydrolyzing a hydrolyzable organic titanate to precipitate titanium oxide on the surfaces of metal flakes. This process does not give a coloring pigment having a deep color and the color flop property, either.

JP-A-11-80587 discloses a method in which the color flop property is attained by coating flaky aluminum with hydrolyzates of a titanium alcoholate and calcining the coated aluminum at 400°C or higher. According to this method, a deep color or the color flop property cannot be attained in some cases depending on the grade of the flaky aluminum and other components, so that the production of a pigment having a stable quality cannot be assured.

US-A-5,931,996 discloses a colored aluminum pigment comprising a flake-form aluminum substrate coated with a single layer of metal oxide, derived from a metal acid ester, such as silicic acid tetraethyl ester, wherein the layer of the metal oxide contains at least one color pigment.

### SUMMARY OF THE INVENTION

The present invention provides a flaky aluminum pigment that can have a stable color flop property and regardless of the particle size and change with time of starting flaky aluminum, has a large variety of color tones due to the interference colors, and has a high hiding power. In addition, the present invention relates to a coating composition for obtaining a novel metallic coating film that has an excellent color flop property and is free from aggregation, by utilizing said pigment.

The present inventors earnestly investigated in order to solve the above problems, and consequently found that the object can be achieved by hydrolyzing a material represented by R-O-[Ti(OR)₂-O-]ₙ-R and coating the surface of flaky aluminum with the hydrolyzates, whereby the present invention has been accomplished.

A first aspect of the invention is directed to an aluminum pigment composition comprising flaky aluminum, wherein said flaky aluminum as a base material is coated with a coating layer comprising hydrolyzates (a) of a titanium alcoholate represented by the general formula (1):

R-O-[Ti(OR)₂-O-]ₙ-R (1)

wherein each of the Rs, which may be the same or different, is an alkyl group, and n is an integer of 1 to 40, an aluminum component (b) in a proportion of 0.1 to 20% in terms of aluminum based on the amount of the coating layer, and a component derived from a basic substance (c), in a proportion of 0.001 to 5 mass% based on the mass of the aluminum pigment composition.

A second aspect of the invention is directed to an aluminum pigment composition according to the first aspect of the invention, wherein the main hydrolyzate among the hydrolyzates (a) of the titanium alcoholate is titanium dioxide. In detail, it is preferable that the hydrolyzates (a) of the titanium alcoholate contain 50 mass% or more of titanium dioxide.

A third aspect of the invention is directed to an aluminum pigment composition according to any one of the first or second aspect of the invention, wherein the surface of the coating layer covering the flaky aluminum has convexities with a diameter of not more than 2.0 µm and not less than 0.1 µm.

A fourthaspect of the invention is directed to a process for producing an aluminum pigment composition according to any one of the first to third aspects of the invention, which comprises a step of hydrolyzing a titanium alcoholate represented by the general formula (1):

R-O-[Ti(OR)₂-O-]ₙ-R (1)

wherein each of the Rs, which may be the same or different, is an alkyl group, and n is an integer of 1 to 40, by adding water to a solvent containing said titanium alcoholate and a basic substance (c) and having flaky aluminum dispersed therein and a step of coating said flaky aluminum as a base material with the resulting hydrolyzates.

A fifth aspect of the invention is directed to a coating composition, an ink composition, a resin compound or a cosmetic, which comprises an aluminum pigment composition according to any one of the first to fourth aspects of the invention.

An sixth aspect of the invention is directed to a coated product obtained by coating with the coating composition according to the fifth aspect of the invention, or a print obtained by printing using the ink composition according to the fifth aspect of the invention.

### BRIEF DESCRIPTION OF DRAWINGS

Fig. 1 is an electron micrograph of the surface of the aluminum pigment composition obtained in Example 1 of the present invention.
Fig. 2 is an electron micrograph of the surface of the aluminum pigment composition obtained in Comparative Example 1 of the present invention.

### BEST MODE FOR CARRYING OUT THE INVENTION

The present invention is explained below in further detail.

In the present invention, a titanium alcoholate is hydrolyzed under suitable conditions and flaky aluminum is coated with the hydrolyzates, after which the coated flaky aluminum is preferably dried and then calcined, whereby an aluminum pigment composition having the color flop property can be stably obtained irrespective of the particle size and change with time of the flaky aluminum.

The titanium alcoholate used in the present invention is a compound represented by the general formula (1):

R-O-[Ti(OR)₂-O-]ₙ-R (1)

wherein Ti is a titanium atom, O is an oxygen atom, each of the Rs, which may be the same or different, is an alkyl group, and n is an integer of 1 to 40.

As the aforesaid alkyl group for R, there are used methyl, ethyl, propyl, butyl, octyl, stearyl and the like. Of these, the groups from ethyl to octyl are especially preferable. The reason is as follows. The hydrolysis becomes slower with an increase of the molecular weight of the alkyl group. On the other hand, when the molecular weight is too high, the titanium alcoholate becomes waxy, so that a solvent is limited in which the titanium alcoholate can be uniformly dispersed.

As the titanium alcoholate, there may be used not only a monomer (n = 1 in the general formula (1)) but also a condensate such as a dimer (n = 2), trimer (n = 3), tetramer (n = 4), heptamer (n = 7), decamer (n = 10) or the like. However, when the integer for n is too large, the viscosity of the titanium alcoholate itself is increased, so that its dissolution in a solvent becomes difficult. Therefore, n is preferably 10 or less.

The titanium alcoholate used in the present invention includes, for example, tetramethoxytitanium, tetraethoxytitanium, tetraisopropoxytitanium, tetra-n-butoxytitanium, tetra-sec-butoxytitanium, tetra-tert-butoxytitanium, tetrakis(2-ethylhexyloxy)titanium, tetrastearyltitanium, di-i-propoxy-bis(acetonato)-titanium, di-n-butoxy·bis(triethanolaminato)titanium, titanium-i-propoxyoctylene glycolate, titanium stearate, and condensates thereof.

Of these, tetraisopropoxytitanium, tetra-n-butoxytitanium, and dimers, tetramers, heptamers and decamers of tetra-n-butoxytianium are especially preferable. These titanium alcoholates may be used singly or as a mixture thereof.

The amount of the titanium alcoholate added in the present invention is preferably not more than 360 mg and not less than 1 mg, in terms of the metal, per square meter (the specific surface area) of the flaky aluminum. The amount is preferably not less than 1 mg from the viewpoint of color development. The amount is preferably not more than 360 mg from the viewpoint of the depth of the interference colors and the reduction of suspended particles.

Although the flaky aluminum used in the present invention is not particularly limited in shape, it preferably has a smooth surface and is preferably flaky because interference due to parallel thin films is utilized. For example, flaky aluminum having a thickness in a range of 0.01 to 5 µm and a length or width in a range of 1 to 100 µm is preferable. The aspect ratio is preferably in a range of 10 to 250. Here, the aspect ratio is the ratio between the major axis and thickness of the flaky aluminum. It is also possible to use flaky aluminum obtained by vacuum deposition of aluminum on a film, peeling of the aluminum deposition thin film from the former film, and grinding of the aluminum deposition thin film.

In the present invention, the above-mentioned titanium alcoholate is hydrolyzed in the presence of a basic substance in order to obtain an aluminum pigment composition excellent in the color flop property.

Here, the basic substance refers to a substance that gives a basic aqueous solution. The basic substance includes, for example, basic salts such as oxide salts, hydroxide salts, etc.; basic oxides such as magnesium oxide, calcium oxide, strontium oxide, barium oxide, etc.; hydroxides such as lithium hydroxide, sodium hydroxide, potassium hydroxide, magnesium hydroxide, calcium hydroxide, strontium hydroxide, barium hydroxide, iron hydroxide, etc.; carbonates and hydrogencarbonates of alkali metals, such as potassium carbonate, potassium hydrogencarbonate, sodium carbonate, sodium hydrogencarbonate, etc.; ammonia and ammonium compounds such as ammonium carbonate, ammonium hydrogencarbonate, etc.; morpholine, pyridine, and salts thereof; oxalates such as potassium tetraoxalate, etc.; borates such as sodium tetraborate, potassium borate, ammonium borate, etc.; tris(hydroxymethyl)aminomethane; tris(hydroxymethyl)-aminomethane hydrochloride; alkali metal salts of weak acids; and the following amines.

The amines include primary amines such as methylamine, ethylamine, propylamine, butylamine, amylamine, hexylamine, heptylamine, octylamine, caprylamine, laurylamine, stearylamine, oleylamine, etc.;
secondary amines such as dimethylamine, diethylamine, dipropylamine, diisopropylamine, dibutylamine, diamylamine, distearylamine, etc.;
tertiary amines such as trimethylamine, triethylamine, tripropylamine, tributylamine, triamylamine, dimethyloctylamine, dimethyldecylamine, dimethyllaurylamine, dimethylmyristylamine, dimethylpalmitylamine, dimethylstearylamine, dilaurylmethylamine, trioctylamine, etc.;
unsaturated amines such as allylamine, diallylamine, triallylamine, etc.;
alicyclic amines such as cyclopropylamine, cyclobutylamine, cyclopentylamine, cyclohexylamine, etc.;
aromatic amines such as aniline, methylaniline, dimethylaniline, ethylaniline, diethylaniline, toluidine, benzylamine, dibenzylamine, tribenzylamine, diphenylamine, triphenylamine, naphthylamine, etc.;
diamines such as methylenediamine, ethylenediamine, trimethylenediamine, tetramethylenediamine, pentamethylenediamine, hexamethylenediamine, 1,7-diaminoheptane, 1,8-diaminooctane, 1,9-diaminononane, 1,10-diaminodecane, o-phenylenediamine, p-phenylenediamine, m-phenylenediamine, benzidine, diaminostilbene, tolidine, menthenediamine, isophoronediamine, m-xylenediamine, diaminodiphenylmethane, diaminodiphenylsulfone, etc.; and
polyamines such as diethylenetriamine, triethylenetetramine, tetraethylenepentamine, diethylaminopropylamine, polyamide-polyamines, N-aminoethylpiperazine, bis(4-amino-3-methylcyclohexyl)-methane, bis(4-aminocyclohecyl)methane, etc.

Of the basic substances mentioned above, the alkali metal salts of weak acids and the amines are preferable. Tetraethylenepentamine, triethylenetetramine, diethylenetriamine, sodium tetraborate, sodium carbonate and sodium hydrogencarbonate are more preferable.

A substance having both a hydroxyl group and a basic amino group retards the reaction when it is added to a solvent having flaky aluminum dispersed therein. It is speculated as a reason for this that since the hydroxyl group has a strong tendency to be adsorbed onto the surface of aluminum and as a result the basic group turns outward, the surface of the flaky aluminum is less likely corroded, so that the aluminum is less likely incorporated into the coating layer. Therefore, though the substance having both a hydroxyl group and a basic amino group can be used as a reaction retarder, a substance not having a hydroxyl group and a basic amino group at the same time is preferred in order to accelerate the reaction.

The substance having both a hydroxyl group and a basic amino group includes alkanolamines such as ethanolamine, diethanolamine and triethanolamine.

In the present invention, the layer covering flaky aluminum which contains titanium alcoholate hydrolyzates should contain an aluminum component for the exhibition of an excellent color flop property. This aluminum component is derived from the flaky aluminum to be coated, and can be introduced into the coating layer by carrying out the above-mentioned procedure consisting of hydrolysis of the titanium alcoholate, drying of the flaky aluminum coated and its calcining, by the predetermined method described hereinafter. The content of said aluminum component is preferably 1 ppm to 50%, more preferably 0.1% to 50%, still more preferably 0.1% to 20%. The aluminum component can be subjected to quantitative analysis by a conventional analytical method such as Auger electron spectroscopy. For example, when Auger electron spectroscopy is employed, the change (proportional to the molar concentration) of the concentration (the number of atoms) of each element present in the coating layer can be known in the region from the coating layer surface to the innermost. Therefore, on the basis of this change, the concentration of each element is calculated by integration over the coating layer portion, and the ratio of the this concentration to the total concentration of all detected elements (e.g. aluminum, oxygen and titanium) is calculated, whereby the presence proportion (%) of each element in the coating layer can be known.

In the present invention, although a mechanism by which the aluminum component contained in the coating layer causes the exhibition of the color flop property has not been elucidated, the following is conjectured. Owing to the aluminum component contained in the coating layer, the crystallization of the coating layer does not proceed markedly in the coated flaky aluminum even when the coated flaky aluminum is calcined. Therefore, no remarkable volume shrinkage of the coating layer accompanies the crystallization. Accordingly, the coating layer is formed as a thick layer without cracking, so that a high chromaticity is attained. It is also conjectured that since the aluminum component is contained, convexities with a diameter of not more than 2.0 µm and not less than 0.1 µm are formed on the coating layer surface, resulting in the exhibition of a high color flop property.

With respect to preferable shape of convexities formed on the surface, they have a diameter, in the direction of width, of preferably not more than 2.0 µm and not less than 0.1 µm, more preferably not more than 2.0 µm and not less than 0.5 µm; a maximum height of preferably not less than 40 nm, more preferably not less than 50 nm, further preferably not less than 60 nm, and preferably not more than 500 nm, more preferably not more than 300 nm; an average height of preferably not less than 7 nm, more preferably not less than 10 nm, and preferably not more than 100 nm, more preferably not more than 50 nm; and a standard deviation of height of preferably not less than 13 nm, more preferably not less than 20 nm, and preferably not more than 100 nm, more preferably not more than 50 nm.

In the present invention, the basic substance is used in the production process in order to incorporate the aluminum component into the layer covering the flaky aluminum which contains titanium alcoholate hydrolyzates. It was found that owing to this use, one or more components derived from the basic substance used, such as a portion of the basic substance or pyrolyzates of the basic substance also remain in the coating layer. For example, when an alkali metal salt of a weak acid is used as the basic substance, the metal remains in the coating layer. When an amine is used as the basic substance, nitrogen compounds produced by pyrolysis of the basic substance remain in the coating layer. Specifically, when a sodium salt of a weak acid is used, sodium remains. When tetraethylenepentamine is used, pyrazine and butylpyrazine remain. As to a method for quantifying the remaining substance(s), a fluorescent X-ray analysis apparatus may be used when a metal remains. When an amine is used as the basic substance, pyrolysis gas chromatography is employed in which the amine is heated at a temperature higher than the calcining temperature, and the resulting gas is analyzed.

The following is conjectured. Since the coating layer contains not only the aluminum component but also the component(s) derived from the basic substance, the crystallization of the coating layer is further inhibited in the coated flaky aluminum even when the coated flaky aluminum is calcined. Therefore, no volume shrinkage of the coating layer accompanies the crystallization. Accordingly, the coating layer is formed as a thick layer without cracking, so that a high chromaticity is attained. It is also conjectured that since the aluminum component and the component(s) derived from the basic substance are contained, convexities with a diameter of not more than 2.0 µm and not less than 0.1 µm are formed on the coating layer surface, resulting in the exhibition of the color flop property.

The content of the component(s) derived from the basic substance is preferably not more than 5 mass% and not less than 10 ppm, more preferably not more than 3 mass% and not less than 0.01 mass%. The content is preferably not less than 10 ppm from the viewpoint of the synergistic effect on the exhibition of the color flop property. The content is preferably not more than 5 mass% from the viewpoint of long-term stability.

By the hydrolysis of the titanium alcoholate in a solvent containing the basic substance and flaky aluminum dispersed in the solvent, the basic substance is adsorbed on the surface of the flaky aluminum together with hydrolyzates of the titanium alcoholate. Therefore, the aluminum component could be uniformly incorporated into the coating layer irrespective of the surface profile of the flaky aluminum, for example, the thicknesses of organic substances remaining on the surface and an oxide layer. Accordingly, a high chromaticity and a high color flop property could be attained irrespective of the grade, particle size and change over time of the flaky aluminum used as a starting material.

The hydrolysis of the titanium alcoholate in the present invention can be carried out by suspending flaky aluminum in a suitable solvent, and adding thereto the titanium alcoholate, i.e., a compound represented by the general formula (1):

R-O-[Ti(OR)₂-O-]ₙ-R (1),

water in an amount equal to or larger than that stoichiometrically required for hydrolyzing said titanium alcoholate, and the basic substance. For example, when n = 1 in the general formula (1), the reaction:

Ti(OR)₄ + 2H₂O → TiO₂ + 4 ROH

takes place. It is conjectured that titanium oxide, titanium hydroxide and a compound containing the remaining alkyl group are produced by the hydrolysis and are adsorbed on the surface of the flaky aluminum.

Although the solvent used in the hydrolysis is not particularly limited, it is preferable to use a solvent capable of dissolving the titanium alcoholate, such as a alcohol, propylene glycol, ethylene glycol, mineral spirit, solvent naphtha, benzene, toluene, xylene, petroleum benzine, ethyl acetate, butyl acetate or the like. These may be used singly or as a mixture thereof. Of these, the same alcohol as that produced as a by-product by the hydrolysis of the titanium alcoholate is more preferably used as the solvent.

Methods for adding the flaky aluminum, the titanium alcoholate, water and the basic substance in the above-mentioned hydrolysis are explained below.

First, the flaky aluminum may be used as it is in the form of powder, or it may be used after being made into a paste containing the flaky aluminum and 15% to 60% of a solvent such as mineral spirits, solvent naphtha, toluene, butyl acetate or the like, in order to facilitate its handling. In either case, the flaky aluminum is preferably dispersed in the solvent for hydrolysis at first.

The titanium alcoholate may be added after being reduced in concentration by dilution with a solvent. Other metal alcoholates such as zirconium alcoholate, aluminum alcoholate, alkoxysilanes, etc. may be mixed with the titanium alcoholate.

In the above-mentioned hydrolysis of the titanium alcoholate, water should be added in an amount equal to or larger than a minimum amount stoichiometrically required for carrying out the hydrolysis. When the amount of water added is too small, the reaction requires too much time, or the titanium alcoholate tends to be condensed into a straight chain and hence does not undergo three-dimensional crosslinking on the surface of the flaky aluminum, so that a uniform film is hardly formed. On the other hand, when too much water is added, gelatinization tends to occur, so that the flaky aluminum is aggregated. Moreover, water is likely to react with the flaky aluminum to produce hydrogen gas. The amount of water added is preferably not more than 100 times and not less than 10 times the number of moles of the titanium alcoholate.

In general, the above-mentioned titanium alcoholate and water are added to a solvent containing the flaky aluminum dispersed therein. Each of them may be added to the solvent all at once, or may be added stepwise in small portions. As to the order of their addition, it is possible to dissolve or suspend the titanium alcoholate in the solvent at first and then add water thereto, or it is possible to dissolve or suspend water in the solvent at first and then add the titanium alcoholate thereto. Alternatively, they may be added in small portions at the same time or alternately.

It can be speculated that hydrolyzates of the titanium alcoholate and the flaky aluminum are bonded to each other through hydroxyl groups present on the flaky aluminum surface. When the bonding power is so weak that a large number of suspended particles are produced, there may be added a compound having at each end a group capable of binding to the hydrolyzates of the titanium alcoholate and the flaky aluminum through a hydrogen bond or an ionic bond (e.g. a dicarboxylic acid, an unsaturated carboxylic acid or a silane coupling agent) as an anchor site.

The amount of the basic substance added may be properly determined depending on the kind of the basic substance and the kind and particle size of the flaky aluminum. However, when the amount is too small, the addition is ineffective. When the amount is too large, the flaky aluminum is corroded, or the hydrolysis of the titanium alcoholate becomes abnormal, so that the flaky aluminum is not coated. The amount is preferably not more than 10 mass% and not less than 0.1 mass%, more preferably not more than 5 mass% and not less than 0.1 mass%, still more preferably not more than 5 mass% and not less than 0.5 mass%, based on the mass of the flaky aluminum.

The basic substance may be added directly to a solvent containing the flaky aluminum dispersed therein, or it may be added to a solvent together with the flaky aluminum after their previous and thorough mixing. The basic substance may be dispersed in a solvent after being thoroughly mixed with a paste prepared by mixing the flaky aluminum with a solvent. The basic substance may be previously dissolved in water that is added for hydrolyzing the titanium alcoholate.

A neutral salt such as sodium chloride, potassium chloride, magnesium chloride, calcium chloride, sodium azide or the like and an alkali metal salt of a weak acid as the basic substance may be used in combination. This combination permits reduction of the amount of the basic substance added and attainment of both a high chromaticity and the color flop property.

The hydrolysis proceeds even at room temperature. When the reaction is too rapid, cooling may be conducted. When the acceleration of the reaction is desired, heating may be conducted. The hydrolysis may be carried out usually in a range of not higher than 80°C and not lower than 10°C. The hydrolysis is preferably carried out, in particular, at a temperature not higher than 50°C and not lower than 30°C.

If necessary, a method different from the above general hydrolysis method may be adopted.

For example, for obtaining a denser layer covering the flaky aluminum, it is advantageous to allow the hydrolysis to proceed slowly. Therefore, in this case, it is preferable to add water in small portions to a solvent containing the flaky aluminum, basic substance and titanium alcoholate dispersed or dissolved therein. In this case, water may be added alone in small portions, or a dilution of water with a hydrophilic solvent such as an alcohol may be added in small portions.

In addition, the flaky aluminum may be coated with multiple layers of, for example, titanium oxide by repeating coating treatment of the flaky aluminum using the titanium alcoholate, several times. The flaky aluminum can be coated with multiple layers by also using other metal alcoholates such as zirconium alcoholate, aluminum alcoholate, alkoxysilanes, etc.

The thus obtained flaky aluminum coated with hydrolyzates of the titanium alcholate is separated from the solvent by filtration to obtain the aluminum pigment composition of the present invention. The aluminum pigment composition is preferably obtained as a composition having a higher depth of color by calcining the coated and dried flaky aluminum at a temperature not higher than the melting point of the flaky aluminum and not lower than 250°C, more preferably 400°C. Although the calcining may be conducted under an air atmosphere, it is preferably conducted while introducing carbon dioxide or an inert gas such as nitrogen or argon for safety.

By the above production process, an aluminum pigment composition having various color tones due to interference colors and a high hiding power can be obtained. Moreover, the utilization of said composition as a pigment for coating material has made it possible to obtain coating films, prints, molded articles and the like, which have the color flop property, are free from aggregation of the pigment and have a novel metallic appearance.

Coating (including ink) compositions using the aluminum pigment composition of the present invention can be used as solvent type coating materials, water-borne coating materials, powder coating materials, etc. These coating materials are composed mainly of two basic components, i.e., a coating resin and the aluminum pigment composition. The solvent type coating materials and the water-borne coating materials further contains a diluent as a third component.

As the coating resin used in the solvent type coating materials, any coating resin conventionally used in metallic coating materials may be used. The resin includes, for example, acrylic resins, alkyd resins, oil-free alkyd resins, vinyl chloride resins, vinyl chloride-vinyl acetate copolymers, ethylene-vinyl acetate copolymer resins, urethane resins, melamine resins, unsaturated polyester resins, urea resins, cellulosic resins, epoxy resins, silicone resins, coumarone resins, coumarone-indene resins, xylene resins, phenolic resins, ketone resins, and fluororesins. These may be used singly or as a mixture thereof.

Particularly when the solvent type coating material is used as a finish coating material for automobile, it is possible to use, for example, a combination of resins such as a polymer having acid groups and a polymer having epoxy groups, as a countermeasure against acid rain, which is a recent problem.

A combination of an acrylic resin and a melamine resin is most generally used in the coating composition of the present invention. In this case, the melamine resin functions like a crosslinking agent for the acrylic resin. As the acrylic resin, there are used those obtained by using a combination of acrylic acid and a derivative monomer thereof (e.g. methyl acrylate or ethyl acrylate) or a combination of methacrylic acid and a derivative monomer thereof (e.g. methyl methacrylate or ethyl methacrylate) as a main component, and copolymerizing therewith an acrylamide, acrylonitrile, styrene-vinyltoluene, vinyl acetate or the like if necessary. As the melamine resin, methylolmelamine, methylated methylolmelamine, butylated methylolmelamine, etc. are used. As a resin used in combination with the acrylic resin, polyester resins, alkyd resins, fluororesins, etc. are usually used.

The amount of the aluminum pigment composition of the present invention used in the solvent type coating material is 0.1 part by mass to 100 parts by mass per 100 parts by mass of the coating resin. The aluminum pigment composition is preferably used in an amount of, in particular, 1 part by mass to 50 parts by mass. The amount is preferably 0.1 part by mass or more from the viewpoint of coloring effect, and is preferably 100 parts by mass or less from the viewpoint of coating efficiency and physical properties of a coating film. If necessary, the aluminum pigment composition may be previously made into a paste with a diluent for the solvent type coating material, in order to facilitate the dispersion of the composition.

The diluent used in the solvent type coating material includes aromatic compounds such as toluene, xylene, etc.; aliphatic compounds such as hexane, cyclohexane, heptane, octane, mineral spirits, etc.; alcohols such as methanol, ethanol, 1-propanol, 2-propanol, 1-butanol, 2-butanol, etc.; esters such as ethyl acetate, n-propyl acetate, isopropyl acetate, butyl acetate, isobutyl acetate, etc.; ketones such as acetone, methyl ethyl ketone, methyl isobutyl ketone, etc.; chlorine compounds such as trichloroethylene, etc.; and Cellosolves such as ethylene glycol monoethyl ether, ethylene glycol monomethyl acetate, ethylene glycol monoethyl ether acetate, ethylene glycol monobutyl ether, propylene glycol monomethyl ether, dipropylene glycol monomethyl ether, etc. These diluents may be used singly or as a mixture thereof. Their combination is determined in view of dissolving properties for the coating resin, film-forming properties, coating efficiency, etc.

The solvent type coating material may be incorporated with additives such as flaky pigments, coloring pigments, dyes, photochromic substances, lubricants, wetting agents, dispersants, segregation-preventing agents, leveling agents, slip agents, anti-skinning agents, anti-gelling agents, defoaming agents, curing catalysts, ultraviolet absorbers, antioxidants, surface-treating agents, anti-sagging agents, thickening agents, microgels, etc., which are generally used in the coating material field.

Specific examples of materials related to, in particular, the coloring referred to herein are given below. The flaky pigments include flaky aluminum, lamellar iron oxide, phthalocyanine flakes, graphite, mica coated with titanium dioxide, colored mica, etc. Coloring pigments include azo lake pigments, phthalocyanine pigments, indigo pigments, perinone pigments, perylene pigments, quinophthalone pigments, dioxazine pigments, quinacridone pigments, isoindolinone pigments, metal complex pigments, chrome yellow, yellow iron oxide, red iron oxide, carbon black, titanium dioxide, etc. The dyes include azo dyes, anthraquinone dyes, indigoid dyes, sulfur dyes, triphenylmethane dyes, pyrazolone dyes, stilbene dyes, nitro dyes, etc. The photochromic substances include azobenzenes, spiropyrans, spirooxazines, phenothiazines, etc.

In addition, the lubricants and the dispersants are also factors related to the coloring because the orientation and the like of the pigment in the coating composition affect the color tone. The lubricants include, for example, fatty acid lubricants such as stearic acid, palmitic acid, oleic acid, linoleic acid and linolenic acid. The dispersants include polyacrylic acid partial alkyl esters, polyalkylene-polyamines, condensation products of a naphthalenesulfonate and formaldehyde, polystyrene sulfonic acid salts, polyacrylic acid salts, salts of copolymers of a vinyl compound and a carboxylic acid type monomer, carboxymethyl cellulose, and poly(vinyl alcohol)s. The lubricants and the dispersants may be used by adopting a method for providing them directly on the surface of the pigment in the composition of the present invention, or they may be incorporated into the composition. Further, in order to improve the adhesion of the coated film and weather resistance, the surface of pigments in the aluminum pigment composition of the present invention may be subjected to a known surface treatment conventionally carried out for titanium oxide and pearl mica pigments. The surface may be treated or coated with, for example, a metal oxide or metal oxide hydrate of aluminum, silicon, titanium, zirconium, tin, zinc or the like, a polyol such as trimethylolpropane, an organic substance such as silicone resins, or silane-, titanate-, aluminum-based coupling agent.

Particularly when a mixture of a pearl mica pigment and the aluminum pigment composition of the present invention in a proper ratio is used, or when a proper coating film structure is employed (for example, a pearl mica layer is formed on a layer containing the metallic pigment), not only is there interference colors having a metallic appearance but also interference colors which create soft reflected light are observed. Therefore, the depth of color is increased and a larger decorative effect can be obtained.

Particularly when the aluminum pigment composition of the present invention and a pearl mica pigment are used in the finish coating film of an automobile, the particle size of the aluminum pigment composition of the present invention is adjusted to be in a range of 1 to 50 µm, preferably 5 to 40 µm, more preferably 5 to 20 µm, and the particle size of the pearl mica pigment is adjusted to be in a range of 1 to 90 µm, preferably 5 to 40 µm. The sharper the particle size distribution of each of the pigments, the higher the chromaticity.

The coating composition containing the aluminum pigment composition of the present invention can be used as a water-borne coating material. In this case, the production of hydrogen gas by the reaction of the metal with water should be prevented by reducing the water content of the coating material or adding a reaction inhibitor such as a phosphoric ester. As the resin for the water-borne coating material, watersoluble resins or water-dispersible resins can be used singly or as a mixture thereof.

The kind of the resin is not limited and is varied greatly depending on its purpose of use. In general, the resin includes, for example, acrylic resins, amide group-containing acrylic resins, acrylic/ cellulose acetate butyrate mixed resins, epoxy resins, alkyd resins, nitrocellulose resins, polyamide resins, polyester resins, polyurethane resins, epoxy-modified alkyd resins, and the above-exemplified resins for a countermeasure against acid rain. These resins may be used singly or in combination with a curing agent such as a melamine resin, urea resin, isocyanate compound, blocked isocyanate compound or the like.

The amount of the aluminum pigment composition of the present invention used in the water-borne coating material is 0.1 part by mass to 100 parts by mass per 100 parts by mass of the coating resin. The aluminum pigment composition is preferably used in an amount of, in particular, 1 part by mass to 50 parts by mass. The amount is preferably 0.1 part by mass or more from the viewpoint of coloring effect, and is preferably 100 parts by mass or less from the viewpoint of coating efficiency and physical properties of a coating film.

Various additives usually usable in the art, such as the above-exemplified additives, other organic solvents, water, etc. may be added so long as they and their adding amount do not lessen the effect intended according to the present invention.

The coating composition obtained by the use of the aluminum pigment composition of the present invention can be used as a powder coating material. For obtaining a coating film excellent in appearance and the like, the following is possible: an insulating material such as a silicon compound is adhered to the aforesaid aluminum pigment composition; a coating film of the insulating material is formed on the aforesaid aluminum pigment composition; the aforesaid aluminum pigment composition is previously coated with a resin; or another coloring pigment is adhered to the aforesaid aluminum pigment composition, followed by coating with a resin.

The resin used in the powder coating material includes acrylic resins, alkyd resins, polyester resins, polyurethane resins, poly(vinyl acetate) resins, poly(vinyl chloride) resins, epoxy resins, nitrocellulose resins, fluororesins, etc.

The proportion of the aluminum pigment composition is suitably 0.1 to 100 parts by mass, preferably 1 to 50 parts by mass, per 100 parts by mass of coating resin powder. The proportion is preferably 0.1 part by mass or more from the viewpoint of decorative effect, and is preferably 100 parts by mass or less from the viewpoint of physical properties of a coating film.

As a substrate to be coated in the present invention, there may be mentioned metals including iron, aluminum, copper, or alloys thereof; inorganic materials such as glass, cement, concrete, paper, paperboard, cloth, hide, etc.; plastic materials including molded articles of resins such as polyethylenes, polypropylenes, polystyrenes, ethylene-vinyl acetate copolymers, acrylonitrile-styrene copolymers, acrylonitrile-butadiene-styrene copolymers, polyamides, polyacrylates, polyesters, ethylene-polyvinyl alcohol copolymers, vinyl chloride resins, vinylidene chloride resins, polycarbonates, polyurethanes, etc., and various FRPs; woods; and fibrous materials. These substrates to be coated may be previously subjected to a proper undercoating or precoating treatment.

Using the coating (including ink) composition of the present invention, coating, printing or the like can be conducted by a well-known coating method, printing method or the like, such as spray coating, brushing, dip coating, roll coating, curtain coating, letterpress printing, lithography, intaglio printing, gravure printing, screen printing, stencil printing or the like.

For example, in coating an automobile, undercoating and intercoating are usually conducted by the use of an electrodeposition coating material or the like after surface conversion treatment, and after curing of the resulting coating film, finish coating is conducted. In this case, coating operations are carried out by air spray coating or electrostatic coating with an atomization coating machine. The finish coating film is usually composed of a base coat and a top coat formed thereon. The coating composition of the present invention is usually used as the aforesaid base coat.

A coating film as the base coat is preferably formed so as to have a dry film thickness in a range of 10 to 25 µm. The thickness is preferably 10 µm or more from the viewpoint of hiding power for the substrate and the suppression of mottling. Then, a clear-coating material for finish coating is applied as a top coat on the base coat. The coating composition of the present invention can be used without any problem because the application of a clear-coating layer on the base coating film formed by the use of the coating composition does not deteriorate the interference colors. The composition of the clear coating material is not particularly limited though that usually employed in combination with a base coating material is used. In addition, in order to obtain a decorative effect, a coating film may be formed by the use of the pigment of the present invention on a coating film containing a well-known coloring pigment or dye. Alternatively, a coating film containing a well-known coloring pigment or dye may be formed on a coating film formed by the use of the pigment of the present invention.

A molded article having a novel decorative-ness can be developed by kneading a resin with the coating composition using the aluminum pigment composition of the present invention. This resin material is not particularly limited. Any resin may be used as the resin material so long as it can be subjected to conventional molding such as injection molding, transfer molding or the like.

The aluminum pigment composition of the present invention can be used in coating materials for automobiles, bicycles, toys, household electric appliances, buildings, vehicles, industrial machines and their parts, etc., various inks (e.g. printing ink, writing ink, duplicating ink and specialty ink), resin compounds, ceramic products, cosmetics, etc.

Embodiments of the present invention are concretely explained below with examples. The scope of the present invention, however, is not limited by these concrete embodiments.

### (1) Production of a metallic coated plate

According to the recipe for a coating material shown in Table 1, a coating composition was prepared by stirring and mixing the ingredients in a paint shaker for 30 minutes.

The coating composition obtained was applied on an aluminum plate (1 × 70 × 150 mm) to a thickness of 15 µm by air spray coating. After the aluminum plate was allowed to stand at room temperature for 30 minutes, a top clear-coating material having the composition shown in table 2 was applied thereon to a thickness of 35 µm.

After standing at room temperature for 1 hour, the coating films were cured by heating at 140°C for 30 minutes to obtain a metallic coated plate.

**Table 1**

| Recipe for coating material (parts by mass) | |
|---|---|
| Colored flaky aluminum pigment | 3 |
| Thinner *1 | 72 |
| Coating acrylic resin *2 | 32 |
| Coating melamine resin *3 | 8 |

| | |
|---|---|
| *1 : toluene/ethyl acetate/butyl Cellosolve = 7/2/1. *2 : ACRYDIC 47-712 mfd. by Dainippon Ink and Chemicals Inc. *3 : SUPER-BECKAMIN J-820 mfd. by Dainippon Ink and Chemicals Inc. | |

**Table 2**

| Composition of top clear-coating material | |
|---|---|
| (parts by mass) | |
| Xylene | 220 |
| Coating acrylic resin *1 | 200 |
| Coating melamine resin | 50 |

| | |
|---|---|
| *1 : ACRYDIC 44-179 mfd. by Dainippon Ink and Chemicals Inc. | |

### (2) Evaluation of color tone

The color tone and color flop property of the metallic coated plate were evaluated by 1) visual comparison between colors in the highlight direction and shade direction, and on the basis of 2) the difference between values of a hue (h) at an angle of 20° to the regularly reflected light and a hue (h) at an angle of 45° to the regularly reflected light which had been measured with a multi-angle spectrophotometer (CE-740, mfd. by Macbeth Co.) that permits measurement at multiple angles. The depth of color was evaluated on the basis of the value of chromaticity (c*) at an angle of 20° to the regularly reflected light which had been measured with the same measuring instrument as above.

### Example 1

In 512 g of 1-butanol was dispersed 65.8 g of an aluminum paste (MG-01, mfd. by Asahi Kasei Metals Co., Ltd.; heating residue 76%, average particle size ca. 30 µm, thickness ca. 0.7 µm and aspect ratio ca. 43), followed by adding thereto 1.0 g of tetraethylenepentamine and 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the stirring was continued at a temperature of 40°C for another 1 hour. Then, the slurry thus obtained was filtered by suction by the use of a Buchner funnel and filter paper to collect an aluminum pigment composition, which was dried in a hot-air dryer at 100°C for 3 hours.

The aluminum pigment composition was golden. Subsequently, the aluminum pigment composition was calcined at 400°C for 15 minutes to obtain an aluminum pigment composition having remarkable yellowish-green bright points. Using the obtained aluminum pigment composition, a metallic coated plate was produced according to the method described above, and its color flop property was evaluated and found to be so high that the highlight was golden and the shade was light-blue. The result of chromaticity measurement with the multi-angle spectrophotometer was as follows: h = 87 at 20° and h = 243 at 45°. These h values were different as in the case of the relationship between complementary colors and backed up the result of the visual judgment. The chromaticity (c*) was 19, namely, a deep color could be obtained as a golden color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum in substantially the same amount as that of titanium were present in a region from the vicinity of the surface of the titanium alcoholate hydrolyzate layer to the boundary surface between the titanium alcoholate hydrolyzate layer and flaky aluminum. The content of the aluminum component (b) in the coating layer was calculated to be 18%.

The obtained aluminum pigment composition was subjected to pyrolysis gas chromatography in which the composition was heated at 400°C and the resulting gas was analyzed. As a result, pyrazine and butylpyrazine, which were nitrogen compounds and were components derived from tetraethylenepentamine, were detected in amounts of 157 ppm and 171 ppm, respectively, relative to the aluminum pigment composition.

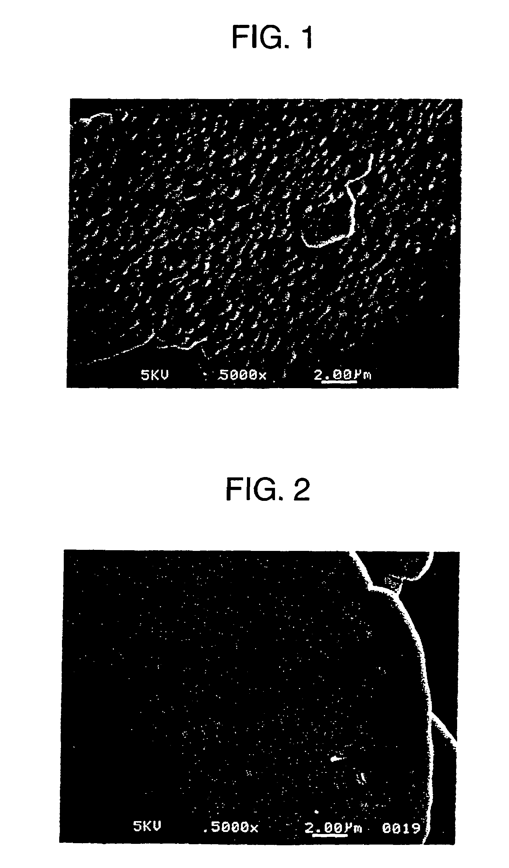

The surface of the obtained aluminum pigment composition was observed by a scanning electron microscope to find that the surface was not smooth but had a large number of convexities with a diameter of about 0.5 µm. The observation result is shown in Fig. 1. The height of the convexities was measured by means of an atomic force microscope. The average height was 13.9 nm, the standard deviation of height 25.5 nm and the maximum height of the convexities 82 nm.

### Example 2

In 512 g of 1-butanol was dispersed 40.6 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 0.5 g of tetraethylenepentamine and 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the stirring was continued at a temperature of 40°C for another 40 minutes. Then, the slurry thus obtained was filtered by suction by the use of a Buchner funnel and filter paper to collect an aluminum pigment composition, which was dried in a hot-air dryer at 100°C for 3 hours.

This aluminum pigment composition was also golden. Subsequently, the aluminum pigment composition was calcined at 400°C for 15 minutes to obtain an aluminum pigment composition having remarkable yellowish-green bright points. Using the obtained aluminum pigment composition, a metallic coated plate was produced according to the method described above, and its color flop property was evaluated and found to be so high that the highlight was golden and the shade was light-blue. The result of chromaticity measurement with the multi-angle spectrophotometer was as follows: h = 89 at 20° and h = 245 at 45°. Thus, the color tone difference was great and backed up the result of the visual judgment. The chromaticity (c*) was 21, namely, a deep color could be obtained as a golden color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum were present in a region from the middle of the titanium alcoholate hydrolyzate layer to the boundary surface between the titanium alcoholate hydrolyzate layer and flaky aluminum. The content of the aluminum component (b) in the coating layer was calculated to be 10%.

The obtained aluminum pigment composition was subjected to pyrolysis gas chromatography in which the composition was heated at 400°C and the resulting gas was analyzed. As a result, pyrazine and butylpyrazine, which were nitrogen compounds and were components derived from tetraethylenepentamine, were detected in amounts of 165 ppm and 196 ppm, respectively, relative to the aluminum pigment composition.

The surface of the obtained aluminum pigment composition was observed by a scanning electron microscope to find that the surface was not smooth but had a large number of convexities with a diameter of about 0.5 µm.

### Example 3

In 512 g of 1-butanol was dispersed 50.6 g of an aluminum paste (MG-01, mfd. by Asahi Kasei Metals Co., Ltd.; heating residue 76%, average particle size ca. 30 µm, thickness ca. 0.7 µm and aspect ratio ca. 43), followed by adding thereto 0.75 g of tetraethylenepentamine and 22.0 g of a tetra-n-butoxytitanium decamer (B-10, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the stirring was continued at a temperature of 40°C for another 1.5 hours. Then, the slurry thus obtained was filtered by suction by the use of a Buchner funnel and filter paper to collect an aluminum pigment composition, which was dried in a hot-air dryer at 100°C for 3 hours.

This aluminum pigment composition was yellowish-green. Subsequently, the aluminum pigment composition was calcined at 400°C for 15 minutes to obtain an aluminum pigment composition having remarkable green bright points. Using the obtained aluminum pigment composition, a metallic coated plate was produced according to the method described above and its color flop property was evaluated to find that the highlight was green and the shade was golden. The result of measurement with the multi-angle spectrophotometer was as follows: h = 118 at 20° and h = 88 at 45°. Thus, the color tone difference was great and backed up the result of the visual judgment. The chromaticity (c*) was 16, namely, a deep color could be obtained as a yellowish-green color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum in substantially the same amount as that of titanium were present in a region from the vicinity of the surface of the titanium alcoholate hydrolyzate layer to the boundary surface between the titanium alcoholate hydrolyzate layer and flaky aluminum. The content of the aluminum component (b) in the coating layer was calculated to be 17%.

The obtained aluminum pigment composition was subjected to pyrolysis gas chromatography in which the composition was heated at 400°C and the resulting gas was analyzed. As a result, pyrazine and butylpyrazine, which were nitrogen compounds and were components derived from tetraethylenepentamine, were detected in amounts of 188 ppm and 221 ppm, respectively, relative to the aluminum pigment composition.

The surface of the obtained aluminum pigment composition was observed by a scanning electron microscope to find that the surface was not smooth but had a large number of convexities with a diameter of about 0.5 µm.

### Example 4

In 512 g of 1-butanol was dispersed 65.8 g of an aluminum paste (MG-01, mfd. by Asahi Kasei Metals Co., Ltd.; heating residue 76%, average particle size ca. 30 µm, thickness ca. 0.7 µm and aspect ratio ca. 43), followed by adding thereto 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of an aqueous sodium tetraborate solution having a concentration of 0.01 mol/kg was added thereto at a rate of 1 g/min with a metering pump. After completion of the addition of the aqueous solution, the stirring was continued at a temperature of 40°C for another 1 hour. Then, the slurry thus obtained was filtered by suction by the use of a Buchner funnel and filter paper to collect an aluminum pigment composition, which was dried in a hot-air dryer at 100°C for 3 hours.

The aluminum pigment composition was golden. Subsequently, the aluminum pigment composition was calcined at 400°C for 15 minutes to obtain an aluminum pigment composition having remarkable yellowish-green bright points. Using the obtained aluminum pigment composition, a metallic coated plate was produced according to the method described above, and its color flop property was evaluated and found to be so high that the highlight was golden and the shade was light-blue. The result of chromaticity measurement with the multi-angle spectrophotometer was as follows: h = 90 at 20° and h = 240 at 45°. These h values were different as in the case of the relationship between complementary colors and backed up the result of the visual judgment. The chromaticity (c*) was 19, namely, a deep color could be obtained as a golden color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum were present in a region from the middle of the titanium alcoholate hydrolyzate layer to the boundary surface between the titanium alcoholate hydrolyzate layer and flaky aluminum. The content of the aluminum component (b) in the coating layer was calculated to be 18%.

The contents of various elements in the obtained aluminum pigment composition were measured with a fluorescent X-ray analysis apparatus. As a result, sodium derived from the sodium tetraborate added as a basic substance in the production was detected in an amount of 150 ppm relative to the aluminum pigment composition.

The surface of the obtained aluminum pigment composition was observed by a scanning electron microscope to find that the surface was not smooth but had a large number of convexities with a diameter of about 0.5 µm.

### Example 5

In 512 g of 1-butanol was dispersed 40.6 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of an aqueous sodium hydrogencarbonate solution having a concentration of 0.05 mol/kg was added thereto at a rate of 1 g/min with a metering pump. After completion of the addition of the aqueous solution, the stirring was continued at a temperature of 40°C for another 40 minutes. Then, the slurry thus obtained was filtered by suction by the use of a Buchner funnel and filter paper to collect an aluminum pigment composition, which was dried in a hot-air dryer at 100°C for 3 hours.

This aluminum pigment composition was also golden. Subsequently, the aluminum pigment composition was calcined at 400°C for 15 minutes to obtain an aluminum pigment composition having remarkable yellowish-green bright points. Using the obtained aluminum pigment composition, a metallic coated plate was produced according to the method described above and its color flop property was evaluated and found to be so high that the highlight was golden and the shade was light-green. The result of chromaticity measurement with the multi-angle spectrophotometer was as follows: h = 92 at 20° and h = 118 at 45°. Thus, the color tone difference was great and backed up the result of the visual judgment. The chromaticity (c*) was 19, namely, a deep color could be obtained as a golden color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum were present in a region from the middle of the titanium alcoholate hydrolyzate layer to the boundary surface between the titanium alcoholate hydrolyzate layer and flaky aluminum. The content of the aluminum component (b) in the coating layer was calculated to be 8%.

The contents of various elements in the obtained aluminum pigment composition were measured with a fluorescent X-ray analysis apparatus. As a result, sodium derived from the sodium hydrogencarbonate added as a basic substance in the production was detected in an amount of 161 ppm relative to the aluminum pigment composition.

The surface of the obtained aluminum pigment composition was observed by a scanning electron microscope to find that the surface was not smooth but had a large number of convexities with a diameter of about 0.5 µm.

### Example 6

In 512 g of 1-butanol was dispersed 31.2 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, an aqueous solution prepared by dissolving 0.06 g of sodium carbonate, 0.05 g of sodium hydrogencarbonate and 0.06 g of sodium chloride in 120 g of pure water was added thereto at a rate of 1 g/min with a metering pump. After completion of the addition of the aqueous solution, the stirring was continued at a temperature of 40°C for another 120 minutes. Then, the slurry thus obtained was filtered by suction by the use of a Buchner funnel and filter paper to collect an aluminum pigment composition, which was dried in a hot-air dryer at 100°C for 3 hours.

This aluminum pigment composition was yellowish-green. Subsequently, the aluminum pigment composition was calcined at 400°C for 15 minutes to obtain an aluminum pigment composition having remarkable green bright points. Using the obtained aluminum pigment composition, a metallic coated plate was produced according to the method described above, and its color flop property was evaluated and found to be so high that the highlight was green and the shade was light yellow. The result of chromaticity measurement with the multi-angle spectrophotometer was as follows: h = 118 at 20° and h = 96 at 45°. This result backed up the result of the visual judgment. The chromaticity (c*) was 15, namely, a deep color could be obtained as a yellowish-green color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum were present in a region from the middle of the titanium alcoholate hydrolyzate layer to the boundary surface between the titanium alcoholate hydrolyzate layer and flaky aluminum. The content of the aluminum component (b) in the coating layer was calculated to be 10%.

The contents of various elements in the obtained aluminum pigment composition were measured with a fluorescent X-ray analysis apparatus. As a result, sodium derived from the sodium carbonate, sodium hydrogencarbonate and sodium chloride added as basic substances in the production was detected in an amount of 188 ppm relative to the aluminum pigment composition.

The surface of the obtained aluminum pigment composition was observed by a scanning electron microscope to find that the surface was not smooth but had a large number of convexities with a diameter of about 0.5 µm.

### Example 7

In 512 g of 1-butanol was dispersed 40.6 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, an aqueous solution prepared by dissolving 0.46 g of sodium tetraborate and 0.06 g of sodium chloride in 120 g of pure water was added thereto at a rate of 1 g/min with a metering pump. After completion of the addition of the aqueous solution, the stirring was continued at a temperature of 40°C for another 70 minutes. Then, the slurry thus obtained was filtered by suction by the use of a Buchner funnel and filter paper to collect an aluminum pigment composition, which was dried in a hot-air dryer at 100°C for 3 hours.

This aluminum pigment composition was also golden. Subsequently, the aluminum pigment composition was calcined at 400°C for 15 minutes to obtain an aluminum pigment composition having remarkable yellowish-green bright points. Using the obtained aluminum pigment composition, a metallic coated plate was produced according to the method described above, and its color flop property was evaluated and found to be so high that the highlight was golden and the shade was light-blue. The result of chromaticity measurement with the multi-angle spectrophotometer was as follows: h = 92 at 20° and h = 257 at 45°. These h values were different as in the case of the relationship between complementary colors and backed up the result of the visual judgment. The chromaticity (c*) was 20, namely, a deep color could be obtained as a golden color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum were present in a region from the middle of the titanium alcoholate hydrolyzate layer to the boundary surface between the titanium alcoholate hydrolyzate layer and flaky aluminum. The content of the aluminum component (b) in the coating layer was calculated to be 10%.

The contents of various elements in the obtained aluminum pigment composition were measured with a fluorescent X-ray analysis apparatus. As a result, sodium derived from the sodium tetraborate and sodium chloride added as basic substances in the production was detected in an amount of 175 ppm relative to the aluminum pigment composition.

The surface of the obtained aluminum pigment composition was observed by a scanning electron microscope to find that the surface was not smooth but had a large number of convexities with a diameter of about 0.5 µm.

### Example 8

In 512 g of 1-butanol was dispersed 40.6 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 0.25 g of tetraethylenepentamine and 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the stirring was continued at a temperature of 40°C for another 40 minutes. Then, the slurry thus obtained was filtered by suction by the use of a Buchner funnel and filter paper to collect an aluminum pigment composition, which was dried in a hot-air dryer at 100°C for 3 hours.

This aluminum pigment composition was also golden. Subsequently, the aluminum pigment composition was calcined at 500°C for 15 minutes to obtain an aluminum pigment composition having remarkable yellowish-green bright points. Using the obtained aluminum pigment composition, a metallic coated plate was produced according to the method described above, and its color flop property was evaluated and found to be so high that the highlight was golden and the shade was light-blue. The result of chromaticity measurement with the multi-angle spectrophotometer was as follows: h = 89 at 20° and h = 230 at 45°. Thus, the color tone difference was great and backed up the result of the visual judgment. The chromaticity (c*) was 12, namely, a deep color could be obtained as a golden color.

In addition, the layer of titanium alcoholate hydrolyzates, i.e., the coating layer of the obtained aluminum pigment composition was analyzed by Auger electron spectroscopy. As a result, it was found that not only oxygen and titanium but also aluminum were present in a region from the middle of the titanium alcoholate hydrolyzate layer to the boundary surface between the titanium alcoholate hydrolyzate layer and flaky aluminum. The content of the aluminum component (b) in the coating layer was calculated to be 6%.

An attempt was made to subject the obtained aluminum pigment composition to pyrolysis gas chromatography in which the composition was heated at 500°C and the resulting gas was analyzed. However, no gas was produced, namely, no component derived from the basic substance (c) was contained in the aluminum pigment composition of the present example.

The surface of the obtained aluminum pigment composition was observed by a scanning electron microscope to find that the surface was not smooth but had a large number of convexities with a diameter of about 0.5 µm.

### Comparative Example 1

The process of Example 1 was repeated except for adding no basic substance.

In 512 g of 1-butanol was dispersed 65.8 g of an aluminum paste (MG-01, mfd. by Asahi Kasei Metals Co., Ltd.; heating residue 76%, average particle size ca. 30 µm, thickness ca. 0.7 µm and aspect ratio ca. 43), followed by adding thereto 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the slurry thus obtained was filtered by suction by the use of a Buchner funnel and filter paper to collect an aluminum pigment composition, which was dried in a hot-air dryer at 100°C for 3 hours.

The aluminum pigment composition was golden. Subsequently, the aluminum pigment composition was calcined at 400°C for 15 minutes to obtain an aluminum pigment composition having a light and yellowish green color. Using the obtained aluminum pigment composition, a metallic coated plate was produced according to the method described above, and its color flop property was evaluated and found to be so low that the highlight was golden and the shade was also golden. The result of chromaticity measurement with the multi-angle spectrophotometer was as follows: h = 91 at 20° and h = 93 at 45°, namely, the colors at these angles were substantially the same. Thus, the measurement result backed up the result of the visual judgment. The chromaticity (c*) was 7, namely, only a light color could be obtained as a golden color.

The layer of titanium alcoholate hydrolyzates, i.e., the coating layer of the colored powder obtained above was analyzed by Auger electron spectroscopy. As a result, it was found that no aluminum was present in the coating layer.

The contents of various elements in the obtained aluminum pigment composition were measured with a fluorescent X-ray analysis apparatus. As a result, only aluminum, titanium and oxygen were detected without detection of a component derived from a basic substance (c).

An attempt was made to subject the obtained aluminum pigment composition to pyrolysis gas chromatography in which the composition was heated at 400°C and the resulting gas was analyzed. However, no gas was produced, namely, no component derived from a basic substance (c) was contained in the aluminum pigment composition of the present comparative example.

The surface of the colored powder obtained was observed by a scanning electron microscope and found to be substantially smooth. The observation result is shown in Fig. 2. The surface roughness was measured by means of an atomic force microscope. The average height was 5.5 nm, the standard deviation of height 10.7 nm and the maximum height of the convexities 25 nm.

### Comparative Example 2

In 512 g of 1-butanol was dispersed 40.6 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the slurry thus obtained was filtered by suction by the use of a Buchner funnel and filter paper, and the residue was dried in a hot-air dryer at 100°C for 3 hours.

The powder thus obtained was blue. Subsequently, this powder was calcined at 400°C for 15 minutes to obtain colored powder having a light blue color. Using the colored powder obtained, a metallic coated plate was produced according to the method described above, and its color flop property was evaluated and found to be so low that the highlight was light-blue and the shade was also light-blue. These colors themselves were very light. The result of measurement with the multi-angle spectrophotometer was as follows: h = 261 at 20° and h = 265 at 45°, namely, the colors at these angles were substantially the same. Thus, the measurement result backed up the result of the visual judgment. The chromaticity (c*) was 4, namely, only a very light color could be obtained as a blue color.

The layer of titanium alcoholate hydrolyzates, i.e., the coating layer of the colored powder obtained was analyzed by Auger electron spectroscopy. As a result, it was found that no aluminum was present in the coating layer.

The contents of various elements in the obtained aluminum pigment composition were measured with a fluorescent X-ray analysis apparatus. As a result, only aluminum, titanium and oxygen were detected without detection of a component derived from a basic substance.

An attempt was made to subject the obtained aluminum pigment composition to pyrolysis gas chromatography in which the composition was heated at 400°C and the resulting gas was analyzed. However, no gas was produced, namely, no component derived from a basic substance (c) was contained in the aluminum pigment composition of the present comparative example.

The surface of the colored powder obtained was observed by a scanning electron microscope and found to be substantially smooth.

### Comparative Example 3

A metallic coating film was formed by using pearl mica (Pearl Grace 90-30R, mfd. by Nihon Koken Kogyo Co., Ltd.) alone as a pigment. As a result, interference colors and the color flop property were confirmed, but the hiding power was so insufficient that an aluminum plate as a substrate was clearly observed

The surface of the pigment was observed by a scanning electron microscope and found to be substantially smooth.

### Comparative Example 4

A metallic coated plate was produced by using Friend Color F500GL (mfd. by Showa Aluminum Corporation), a commercial green metallic pigment, as a pigment. As a result, a green metallic coating film was obtained, but its color tone was not appreciably different from that obtained merely by blending a conventional green pigment and aluminum. The colors of the highlight and the shade remained green with only a change in their lightness. The result of measurement with the multi-angle spectrophotometer was as follows: h = 143 at 20° and h = 146 at 45°, namely, the colors at these angles were substantially the same. Thus, the measurement result backed up the result of the visual judgment.

### Comparative Example 5

In 512 g of 1-butanol was dispersed 40.6 g of an aluminum paste (GX-50A, mfd. by Asahi Kasei Metals Co., Ltd.; heating residue 74%, thickness ca. 0.5 µm, average particle size ca. 20 µm and aspect ratio ca. 40), followed by adding thereto 0.3 g of acrylic acid generally used for preventing the production of a suspended matter and 23.8 g of a tetra-n-butoxytitanium tetramer (B-4, a trade name, mfd. by Nippon Soda Co., Ltd.) while maintaining the dispersed state of the paste. While maintaining the resulting slurry at a temperature of 40°C and continuing its stirring, 120 g of water was added thereto at a rate of 1 g/min with a metering pump so that the water content of the system might be substantially that required for saturation of 1-butanol in the system with water. After completion of the water addition, the slurry thus obtained was filtered by suction by the use of a Buchner funnel and filter paper, and the residue was dried in a hot-air dryer at 100°C for 3 hours.

The powder thus obtained was light-blue. Subsequently, this powder was calcined at 400°C for 15 minutes to obtain colored powder having a light blue color. Using the colored powder obtained, a metallic coated plate was produced according to the method described above, and its color flop property was evaluated and found to be so low that the highlight was light-blue and the shade was also light-blue. These colors themselves were very light. The result of measurement with the multi-angle spectrophotometer was as follows: h = 265 at 20° and h = 267 at 45°, namely, the colors at these angles were substantially the same. Thus, the measurement result backed up the result of the visual judgment. The chromaticity (c*) was 4, namely, only a very light color could be obtained as a blue color.

The layer of titanium alcoholate hydrolyzates, i.e., the coating layer of the colored powder obtained was analyzed by Auger electron spectroscopy. As a result, it was found that no aluminum was present in the coating layer.

The surface of the colored powder obtained was observed by a scanning electron microscope and found to be substantially smooth.

### Comparative Example 6

The process of Comparative Example 5 was repeated except for using 0.3 g of γ-aminopropyl triethoxy silane, an amino-group-containing silane coupling agent generally used for preventing the production of a suspended matter, in place of 0.3 g of acrylic acid.

The powder thus obtained was light-blue. Subsequently, this powder was calcined at 400°C for 15 minutes to obtain colored powder having a light blue color. Using the colored powder obtained, a metallic coated plate was produced according to the method described above, and its color flop property was evaluated and found to be so low that the highlight was light-blue and the shade was also light-blue. These colors themselves were very light. The result of measurement with the multi-angle spectrophotometer was as follows: h = 270 at 20° and h = 266 at 45°, namely, the colors at these angles were substantially the same. Thus, the measurement result backed up the result of the visual judgment. The chromaticity (c*) was 4, namely, only a very light color could be obtained as a blue color.

The layer of titanium alcoholate hydrolyzates, i.e., the coating layer of the colored powder obtained was analyzed by Auger electron spectroscopy. As a result, it was found that no aluminum was present in the coating layer.

The surface of the colored powder obtained was observed by a scanning electron microscope and found to be substantially smooth.

### Comparative Example 7

The process of Comparative Example 5 was repeated except for using 0.3 g of triethanolamine, an alkanolamine generally used as a hydrolysis retarder, in place of 0.3 g of acrylic acid.

The powder thus obtained was light-blue. Subsequently, this powder was calcined at 400°C for 15 minutes to obtain colored powder having a light blue color. Using the colored powder obtained, a metallic coated plate was produced according to the method described above, and its color flop property was evaluated and found to be so low that the highlight was light-blue and the shade was also light-blue. These colors themselves were very light. The result of measurement with the multi-angle spectrophotometer was as follows: h = 268 at 20° and h = 266 at 45°, namely, the colors at these angles were substantially the same. Thus, the measurement result backed up the result of the visual judgment. The chromaticity (c*) was 4, namely, only a very light color could be obtained as a blue color.

The layer of titanium alcoholate hydrolyzates, i.e., the coating layer of the colored powder obtained was analyzed by Auger electron spectroscopy. As a result, it was found that no aluminum was present in the coating layer.

The aluminum pigment composition obtained was subjected to pyrolysis gas chromatography in which the composition was heated at 400°C and the resulting gas was analyzed. As a result, triethanolamine was detected in an amount of 250 ppm relative to the aluminum pigment composition.

The surface of the colored powder obtained was observed by a scanning electron microscope and found to be substantially smooth.

### Comparative Example 8

The process of Comparative Example 5 was repeated except for using 0.3 g of hydrochloric acid, an acid generally used as a hydrolysis catalyst, in place of 0.3 g of acrylic acid.

The powder thus obtained was light-blue and had no luster. Subsequently, this powder was calcined at 400°C for 15 minutes to obtain colored powder having a light blue color. Using the colored powder obtained, a metallic coated plate was produced according to the method described above, and its color flop property was evaluated and found to be so low that the highlight was light-blue and the shade was also light-blue. These colors themselves were very light and slightly luminous. The result of measurement with the multi-angle spectrophotometer was as follows: h = 269 at 20° and h = 269 at 45°, namely, the colors at these angles were substantially the same. Thus, the measurement result backed up the result of the visual judgment. The chromaticity (c*) was 3, namely, only a very light color could be obtained as a blue color.

The layer of titanium alcoholate hydrolyzates, i.e., the coating layer of the colored powder obtained was analyzed by Auger electron spectroscopy. As a result, it was found that no aluminum was present in the coating layer.

The surface of the colored powder obtained was observed by a scanning electron microscope and found to be substantially smooth.

### Comparative Example 9

The process of Comparative Example 5 was repeated except for using 0.02 g of ethylamine, an amine generally used as a hydrolysis catalyst, in place of 0.3 g of acrylic acid.

The powder thus obtained was light-blue. Subsequently, this powder was calcined at 400°C for 15 minutes to obtain colored powder having a light blue color. Using the colored powder obtained, a metallic coated plate was produced according to the method described above, and its color flop property was evaluated and found to be so low that the highlight was light-blue and the shade was also light-blue. These colors themselves were very light. The result of measurement with the multi-angle spectrophotometer was as follows: h = 268 at 20° and h = 266 at 45°, namely, the colors at these angles were substantially the same. Thus, the measurement result backed up the result of the visual judgment. The chromaticity (c*) was 4, namely, only a very light color could be obtained as a blue color.

The layer of titanium alcoholate hydrolyzates, i.e., the coating layer of the colored powder obtained was analyzed by Auger electron spectroscopy. As a result, it was found that no aluminum was present in the coating layer.

The contents of various elements in the aluminum pigment composition obtained were measured with a fluorescent X-ray analysis apparatus. As a result, only aluminum, titanium and oxygen were detected without detection of a component derived from the basic substance.

An attempt was made to subject the obtained aluminum pigment composition to pyrolysis gas chromatography in which the composition was heated at 400°C and the resulting gas was analyzed. However, no gas was produced, namely, no component derived from the basic substance (c) was contained in the aluminum pigment composition of the present comparative example. It is conjectured that the whole ethylamine was decomposed or evaporated because of its small adding amount.

The surface of the colored powder obtained was observed by a scanning electron microscope and found to be substantially smooth.

### INDUSTRIAL APPLICABILITY

By the use of a coating composition using the aluminum pigment composition of the present invention, a novel metallic coating film can be obtained which has a large variety of color tones due to interference colors, has a high hiding power and is free from aggregation of the pigment. Furthermore, according to the production process of the present invention, an aluminum pigment having stable performance characteristics can be obtained irrespective of the particle size and change over time of the starting flaky aluminum.

## Claims

1. An aluminum pigment composition comprising flaky aluminum, wherein said flaky aluminum as a base material is coated with a coating layer comprising hydrolyzates (a) of a titanium alcoholate represented by the general formula (1):
R-O-(Ti(OR)₂-O-]ₙ-R (1)
wherein each of the Rs, which may be the same or different, is an alkyl group, and n is an integer of 1 to 40, an aluminum component (b) in a proportion of 0.1 to 20% in terms of aluminum based on the amount of the coating layer, and a component derived from a basic substance (c), in a proportion of 0.001 to 5 mass% based on the mass of the aluminum pigment composition.

2. An aluminum pigment composition according to claim 1, wherein the main hydrolyzate (a) is formed from titanium dioxide.

3. An aluminum pigment composition according to claim 1 or 2, wherein the surface of the coating layer covering the flaky aluminum has convexities with a diameter of not more than 2.0 µm and not less than 0.1 µm.

4. A process for producing an aluminum pigment composition according to claim 1 , which comprises a step of hydrolyzing a titanium alcoholate represented by the general formula (1):
R-O-[Ti(OR)₂-O-]ₙ-R (1)
wherein each of the Rs, which may be the same or different, is an alkyl group, and n is an integer of 1 to 40, by adding water to a solvent containing said titanium alcoholate and a basic substance (c) and having flaky aluminum dispersed therein and a step of coating said flaky aluminum as a base material with the resulting hydrolyzates.

5. A coating composition, an ink composition, a resin compound or a cosmetic, which comprises an aluminum pigment composition according to any one of claims 1 to 3.

6. A coated product obtained by coating with the coating composition according to claim 5, or a print obtained by printing using the ink composition according to claim 5.

## Patentansprüche

1. Aluminiumpigmentzusammensetzung, die Aluminiumflocken umfasst, wobei die Aluminiumflocken als Basismaterial mit einer Beschichtungsschicht beschichtet sind, die Folgendes umfasst: Hydrolysate (a) eines Titanalkoholats, das durch die allgemeine Formel (1) dargestellt wird:
R-O-[Ti(OR)₂-O-]ₙ-R (1)
wobei jedes der Rs, die gleich oder verschieden sein können, eine Alkylgruppe ist und n eine ganze Zahl von 1 bis 40 ist, eine Aluminiumkomponente (b) in einem Anteil von 0,1 bis 20% als Aluminium, bezogen auf die Menge der Beschichtungsschicht, und eine Komponente, die von einer basischen Substanz (c) abgeleitet ist, in einem Anteil von 0,001 bis 5 Masse-%, bezogen auf die Masse der Aluminiumpigmentzusammensetzung.

2. Aluminiumpigmentzusammensetzung gemäß Anspruch 1, wobei das Haupthydrolysat (a) aus Titandioxid gebildet ist.

3. Aluminiumpigmentzusammensetzung gemäß Anspruch 1 oder 2, wobei die Oberfläche der Beschichtungsschicht, die die Aluminiumflocken bedeckt, Erhebungen mit einem Durchmesser von nicht mehr als 2,0 µm und nicht weniger als 0,1 µm aufweist.

4. Verfahren zur Herstellung einer Aluminiumpigmentzusammensetzung gemäß Anspruch 1, umfassend einen Schritt des Hydrolysierens eines Titanalkoholats, das durch die allgemeine Formel (1) dargestellt wird:
R-O-[Ti(OR)₂-O-]ₙ-R (1)
wobei jedes der Rs, die gleich oder verschieden sein können, eine Alkylgruppe ist und n eine ganze Zahl von 1 bis 40 ist, durch Zugabe von Wasser zu einem Lösungsmittel, das das Titanalkoholat und eine basische Substanz (c) enthält und in dem Aluminiumflocken dispergiert sind, und einen Schritt des Beschichtens der Aluminiumflocken als Basismaterial mit den resultierenden Hydrolysaten.

5. Beschichtungszusammensetzung, Tintenzusammensetzung, Harzverbindung oder Kosmetik, die eine Aluminiumpigmentzusammensetzung gemäß einem der Ansprüche 1 bis 3 umfasst.

6. Beschichtetes Produkt, das durch Beschichten mit der Beschichtungszusammensetzung gemäß Anspruch 5 erhalten wird, oder Druckerzeugnis, das durch Drucken unter Verwendung der Tintenzusammensetzung gemäß Anspruch 5 erhalten wird.

## Revendications

1. Composition de pigment d'aluminium comprenant de l'aluminium en flocons, dans laquelle ledit aluminium en flocons en tant que matériau de base est revêtu d'une couche de revêtement comprenant des hydrolysats (a) d'un alcoolate de titane représenté par la formule générale (1) :
R-O[Ti(OR)₂-O-]ₙ-R (1)
dans laquelle chacun des R, lesquels peuvent être identiques ou différents, est un groupe alkyle, et n est un nombre entier de 1 à 40, un constituant d'aluminium (b) dans une proportion de 0,1 à 20 % en termes d'aluminium rapportée à la quantité de la couche de revêtement, et un constituant dérivé d'une substance basique (c), dans une proportion de 0,001 à 5 % en masse rapportée à la masse de la composition de pigment d'aluminium.

2. Composition de pigment d'aluminium selon la revendication 1, dans laquelle l'hydrolysat principal (a) est constitué de dioxyde de titane.

3. Composition de pigment d'aluminium selon la revendication 1 ou 2, dans laquelle la surface de la couche de revêtement recouvrant l'aluminium en flocons présente des convexités avec un diamètre d'au plus 2,0 µm et d'au moins 0,1 µm.

4. Procédé de production d'une composition de pigment d'aluminium selon la revendication 1, lequel comprend une étape d'hydrolyse d'un alcoolate de titane représenté par la formule générale (1) :
R-O-[Ti(OR)₂-O-]ₙ-R (1)
dans laquelle chacun des R, lesquels peuvent être identiques ou différents, est un groupe alkyle, et n est un nombre entier de 1 à 40, en ajoutant de l'eau à un solvant contenant ledit alcoolate de titane et une substance basique (c) et présentant de l'aluminium en flocons dispersé dans celui-ci et une étape de revêtement dudit aluminium en flocons comme matériau de base avec les hydrolysats résultants.

5. Composition de revêtement, composition d'encre, composé de résine ou cosmétique, qui comprend une composition de pigment d'aluminium selon l'une quelconque des revendications 1 à 3.

6. Produit revêtu obtenu par revêtement avec la composition de revêtement selon la revendication 5 ou impression obtenue par impression en utilisant la composition d'encre selon la revendication 5.
